(19) **Europäisches Patentamt** / **European Patent Office** / **Office européen des brevets**

(11) **EP 3 498 828 B1**

(12) **EUROPEAN PATENT SPECIFICATION**

(45) Date of publication and mention of the grant of the patent:
**03.11.2021 Bulletin 2021/44**

(21) Application number: **17839505.9**

(22) Date of filing: **08.08.2017**

(51) Int Cl.:
*C12N 7/00* *(2006.01)*　　　*C07K 1/34* *(2006.01)*
*C07K 16/00* *(2006.01)*　　*A61L 2/00* *(2006.01)*
*A61L 2/02* *(2006.01)*　　　*C07K 16/08* *(2006.01)*

(86) International application number:
**PCT/JP2017/028841**

(87) International publication number:
**WO 2018/030437 (15.02.2018 Gazette 2018/07)**

(54) **METHOD FOR TREATING SOLUTION CONTAMINATED WITH PORCINE CIRCOVIRUSES**

VERFAHREN ZUR BEHANDLUNG EINER MIT SCHWEINE-CIRCOVIREN VERUNREINIGTEN LÖSUNG

PROCÉDÉ DE TRAITEMENT DE SOLUTIONS CONTAMINÉES PAR DES CIRCOVIRUS PORCINS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2016 JP 2016156506**

(43) Date of publication of application:
**19.06.2019 Bulletin 2019/25**

(73) Proprietor: **Asahi Kasei Medical Co., Ltd.**
**Tokyo 1000006 (JP)**

(72) Inventors:
 • **YUNOKI, Mikihiro**
  **Tokyo 105-6107 (JP)**
 • **URAYAMA, Takeru**
  **Kobe-shi**
  **Hyogo 650-0047 (JP)**
 • **SAKAI, Kaoru**
  **Kobe-shi**
  **Hyogo 650-0047 (JP)**
 • **MIYABAYASHI, Tomoyuki**
  **Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2012/176876**　　**JP-A- 2004 339 079**
**JP-A- 2015 500 860**

 • YANG B. ET AL: "Porcine circovirus (PCV) removal by Q sepharose fast flow chromatography", BIOTECHNOLOGY PROGRESS, vol. 29, no. 6, 20 September 2013 (2013-09-20), pages 1464-1471, XP055296449, ISSN: 8756-7938, DOI: 10.1002/btpr.1804
 • HONGO-HIRASAKI T. ET AL: "Removal of small viruses (parvovirus) from IgG solution by virus removal filter Planova 20N", JOURNAL OF MEMBRANE SCIENCE, vol. 278, no. 1-2, 6 December 2005 (2005-12-06), pages 3-9, XP024931434, ISSN: 0376-7388, DOI: 10.1016/J.MEMSCI.2005.10.057 [retrieved on 2006-07-05]
 • FURUYA K. ET AL: "Implementation of a 20-nm pore-size filter in the plasma-derived Factor VIII manufacturing process", VOX SANGUINIS, vol. 91, no. 2, 16 May 2006 (2006-05-16), pages 119-125, XP055611557, ISSN: 0042-9007, DOI: 10.1111/j.1423-0410.2006.00792.x
 • Asahi Kasei Bioprocess: "Planova Filters - Virus removal for biotherapeutic products", , 2018, XP055611595, Retrieved from the Internet: URL:https://planova.ak-bio.com/products_se rvices/pdf/Planova%20Brochure_EN.pdf?eid=6 04&aid=3464 [retrieved on 2019-08-07]

(Cont. next page)

• YOKOYAMA T. ET AL.: 'Removal of small non-enveloped viruses by nanofiltration.' VOX SANGUINIS vol. 86, 2004, pages 225 - 229, XP055338899

SEQUENCE LISTING

<110> Asahi Kasei Medical Co., Ltd.

<120> Method for Treating Solution Contaminating Porcine Circovirus

<130> 160029WO01

<150> JP2016-156506
<151> 2016-08-09

<160> 3

<170> PatentIn version 3.5

<210> 1
<211> 23
<212> DNA
<213> Artificial Sequence

<220>
<223> Primer for PCV-1 TM Fed.

<400> 1
agaaaggcgg gaattgaaga tac        23

<210> 2
<211> 18
<212> DNA
<213> Artificial Sequence

<220>
<223> Primer for PCV-1 TM Rev.

<400> 2
cacaccccgc cttcagaa          18

<210> 3
<211> 24
<212> DNA
<213> Artificial Sequence

<220>
<223> TaqMan probe

<400> 3
cgtctttcgg cgccatctgt aacg          24

**Description**

Technical Field

The present invention relates to a method for treating a solution contaminated with porcine circovirus.

Background Art

Measures against viruses contaminating steps for producing blood plasma protein fraction preparations or biopharmaceuticals are detection, inactivation and removal of the viruses.

Examples of a method for inactivating viruses include heat treatment methods and treatment methods using chemical agents (for example, Solvent/Detergent: S/D treatment). These methods are known as virus inactivation methods particularly effective against enveloped viruses such as Human immunodeficiency virus (HIV) and Hepatitis C virus (HCV).

However, non-enveloped viruses such as Human parvovirus B19 (B19) and Hepatitis A virus (HAV) are resistant to the above virus inactivation methods, and thus these methods cannot be expected to exhibit the same degree of effect as that exhibited by the methods against enveloped viruses.

Examples of a method for removing viruses include membrane filtration methods. Such a membrane filtration method involves performing separation procedures based on particle sizes, and thus is considered to be advantageous such that viruses can be removed based only on the sizes regardless of the chemical properties and thermal properties of subjects to be separated. Accordingly, in recent years, membrane filtration methods using virus removal membranes are generally employed. Moreover, a cellulose-based virus removal membrane is made of a hydrophilic natural material, exhibits low adsorption of protein preparations to the membrane, and thus is used in production of wide-ranging preparations.

Examples of parvovirus that is a non-enveloped virus include B19, Minute virus of mice (MVM) and Porcine parvovirus (PPV).

B19 is a linear single-stranded DNA virus belonging to the family Parvoviridae, and the size ranges from about 18 to 24 nm.

Also as a virus removal method with high robustness against parvovirus and the like, a membrane filtration method using a virus removal membrane is wide spread. The basic principle of the membrane filtration method is achieved by setting conditions where a target protein can pass through a virus removal membrane, but viruses are unable to pass through the same because of the pore size of the virus removal membrane to be used.

Patent literature 1 discloses a technique for removal of B19, which comprises adjusting a pH of an amino acid solution to range from 4 to 8, causing the aggregation of B19, and thus allowing the membrane to capture B19.

Furthermore, patent literature 2 discloses a method for removing viruses in a protein solution, which comprises adjusting a pH of the protein solution to be less than pH 6 and the electrical conductivity of the same at 7 mS/cm or less, and retaining the levels, so as to cause the aggregation of B19, and then allowing the protein solution to permeate a hydrophilic membrane having a membrane pore size larger than the sizes of monodispersed viruses.

Porcine Circovirus (hereinafter, may also be referred to as "PCV".) is the smallest virus among currently known viruses and the particle size thereof ranges from 16 to 20.7 nm. PCV belongs to the family Circoviridae, and is classified into PCV1, PCV2 and the like, for example. PCV is a virus having a high risk of contaminating into production steps for biopharmaceuticals, and its contamination case was reported in 2010. PCV is also resistant to low pH treatment and heat treatment. According to studies in Non-Patent Literature 1, only low virus removal performance was obtained such that the removal rates of virus removal membranes in a solution containing monodispersed PCV ranged from 0.1 to 0.7 log in the case of Planova 20N and 1.3 to 1.8 log in the case of Planova 15N, suggesting that viruses cannot be removed using virus removal membranes introduced in many production steps for biopharmaceuticals.

Meanwhile, Non-Patent Literature 1 discloses that PCV can be adsorbed and removed by treatment using an ion exchanger, however, it cannot be said that a method for removing PCV more effectively has been sufficiently examined to date.

WO 2012/176876, HONGO-HIRASAKI T. et al.: Removal of small viruses (parvovirus) from IgG solution by virus removal filter Planova 20N", JOURNAL OF MEMBRANE SCIENCE, vol. 278, no. 1-2, 6 December 2005, pages 3 and 9 and FURUYA K. et al.: "Implementation of a 20-nm pore-size filter in the plasma-derived Factor VIII manufacturing process", VOX SANGUINIS, vol. 91, no. 2, 16 May 2006, pages 119-125, disclose the removal of porcine parvovirus (PPV) with use of Planova 20N as membrane with LRV for PPV of 4 or more. Porcine parvovirus is a small non-enveloped virus of size 18-24 nm.

Citation List

Patent Literature

[Patent literature 1: Japanese Patent Laid-Open No. 2004-339079

Patent literature 2: Japanese Patent Laid-Open No. 2006-151840

Non-Patent Literature

Non-Patent Literature 1: Yan B, et al., Porcine Circovirus (PCV) removal by Q sepharose fast flow chromatography. Biotechnol. Prog. 2013; 29: 1464-1471.

Summary of Invention

Technical Problem

An object to be achieved by the present invention is to provide a method for treating a solution contaminated with porcine circovirus.

Solution to Problem

As a result of intensive studies to achieve the above object, the present inventors have discovered that a treatment method can be provided as a method for treating a solution contaminated with porcine circovirus, whereby a solution contaminated with porcine circovirus, which is adjusted to have a specific pH, is treated with a specific filtration membrane, and thus have completed the present invention.
The present invention is reflected by the appendant claims.

Advantageous Effects of Invention

According to the present invention, a method for treating a solution contaminated with porcine circovirus can be provided.

Brief Description of Drawings

[Figure 1] Figure 1 shows the results of confirming changes in capacity of removing PCV under the conditions of pH3.0 to 8.0 with the use of a 0.30 M glycine buffer in Example 1.
[Figure 2] Figure 2 shows the results of confirming changes in capacity of removing PCV under the conditions of 0.10 to 2.0 M glycine buffers (pH4.0) in Example 2.
[Figure 3] Figure 3 shows the results of confirming the effect of pH on PCV removal under the conditions of 0.10% IgG and 0.10 M glycine in Example 3.
[Figure 4] Figure 4 shows the results of confirming the effect of glycine concentrations on PCV removal under the conditions of 0.10% IgG and pH 6.0 in Example 4.
[Figure 5] Figure 5 shows the results of confirming the effect of IgG concentrations on PCV removal under the conditions of 0.30 M glycine buffer (pH6.0) in Example 5.
[Figure 6] Figure 6 shows the results of confirming IgG recovery rates after filtration with P-15 and P-20 under the conditions of 1.0% IgG-containing 0.30 M glycine (pH6.0) in Example 6.

Description of Embodiments

Hereinafter, embodiments for implementing the present invention (hereinafter, referred to as "the embodiments".) are as described below.
A sample to be subjected to the treatment method of the embodiments is a solution contaminated with PCV. The term "solution contaminated with PCV" as used herein may refer to a solution in which PCV is present, and an example thereof is a solution that can be contaminated with PCV.

A solution that can be contaminated with PCV is not required to be actually contaminated with PCV, and may be a solution that can be contaminated with PCV in the process of preparing the solution. Examples of a solution that can be contaminated with PCV do not preclude a solution that is actually contaminated with PCV.

A solution contaminated with PCV may be, for example, a solution that contains a protein and can be contaminated with PCV, a solution that can be contaminated with PCV but contains no protein, a solution that is contaminated with PCV and contains a protein, or a solution that is contaminated with PCV but contains no protein.

Examples of such a solution contaminated with PCV include body fluids that are *in vivo* liquid components of humans, mammals, and the like. Specific examples of body fluids include, but are not particularly limited to, blood (serum and plasma), saliva, sweat, urine, runny nose, seminal fluid, plasma, lymph, enzyme and tissue fluid.

Solutions contaminated with PCV may be solutions obtained by purifying these body fluids etc., as raw materials, and solutions containing body fluids etc., which are obtained by dilution of body fluids etc.

A solution contaminated with PCV may be medium after cell culture, a culture supernatant after cell culture, or the like. Cells to be used herein may be cells that are used for genetic recombination techniques.

Solutions contaminated with PCV may be solutions obtained by purifying these media etc., as raw materials, or solutions containing media etc., obtained by diluting media etc.

A solution contaminated with PCV may be a vaccine, an antibody drug, a genetically modified drug, and the like prepared via cell culturing. A solution contaminated with PCV may also be a preparation or the like prepared using an enzyme derived from a biological tissue in the production steps.

The solution contaminated with PCV contains a protein at a protein concentration ranging from 0.10 to 1.5 w/v %.

In the embodiments, a solution obtained by purifying a body fluid, a medium, or the like as a raw material contains a protein.

In the embodiments, examples of a protein to be contained in a solution contaminated with PCV include, but are not particularly limited to, proteins such as an antibody, erythropoietin, thrombopoietin, tissue-type plasminogen activator, pro-urokinase, thrombomodulin, antithrombin III, protein C, blood coagulation factor VII, blood coagulation factor VIII, blood coagulation factor IX, blood coagulation factor X, blood coagulation factor XI, blood coagulation factor XII, a prothrombin complex, fibrinogen, albumin, gonadotropic hormone, thyroid-stimulating hormone, epidermal growth factor (EGF), hepatocyte growth factor (HGF), keratinocyte growth factor, activin, a bone morphogenetic factor, stem cell factors (SCF) such as G-CSF and M-CSF, interferon $\alpha$, interferon $\beta$, interferon $\gamma$, interleukin 2, interleukin 6, interleukin 10, interleukin 11, a soluble interleukin 4 receptor, tumor necrosis factor $\alpha$, Dnasel, galactosidase, $\alpha$ glucosidase, glucocerebrosidase, hemoglobin, and transferrin, and partial fragments of these proteins.

In the embodiments, proteins to be contained in solutions contaminated with PCV may be proteins or partial fragments of the proteins, which are prepared by binding chemically or via genetic engineering techniques, a radioisotope, a low-molecular-weight drug, a high-molecular-weight drug, or a different protein or partial fragments of the protein or the like to the above proteins or the partial fragments thereof.

A protein to be contained in a solution contaminated with PCV is preferably an antibody such as immunoglobulin, and IgG is suitable among immunoglobulins.

Specific examples of the solution contaminated with PCV and containing a protein include, but are not particularly limited to, solutions obtained in the production steps for an antibody preparation.

In the step of separating and purifying an antibody, through appropriate combination of the ion concentration, the alcohol concentration, the reaction temperature and the protein concentration of raw-material plasma, the solubility of proteins contained in the raw-material plasma is controlled, and a specific protein is precipitated. After separation of the precipitated protein from the supernatant using a high speed centrifuge, and then the precipitate or the supernatant is collected as required. Through setting conditions differing from the above conditions as appropriate for the thus obtained precipitate or supernatant, another protein is precipitated. As a result of repeating the above steps to remove impurities in sequence from the raw-material plasma, a solution containing a high-purity antibody can be obtained. Any solution obtained in the step for separating and purifying an antibody can be used as a solution contaminated with PCV in the embodiments.

Examples of an antibody include a human antibody, a human-type chimeric antibody, a humanized antibody such as a human-type complementarity determining region (CDR) transplanted antibody, as well as antibody fragments and the like thereof.

An antibody is composed of variable regions (V regions) and constant regions (C regions), wherein a heavy chain variable region is referred to as VH, a light chain variable region is referred to as VL, a heavy chain constant region is referred to as CH, and a light chain constant region is referred to as CL.

The term "human-type chimeric antibody" refers to an antibody composed of antibody VH and VL of a non-human animal and human antibody CH and CL. A human-type chimera-transplanted antibody can be produced by designing and constructing cDNA encoding antibody VH and VL of a non-human animal, inserting each of them to an expression vector for animal cells having cDNA encoding human antibody CH and CL, so as to construct a human-type chimera-transplanted antibody expression vector, and then introducing the vector into animal cells for expression.

Such human-type chimeric antibody CH may be any CH as long as it belongs to human immunoglobulin (hereinafter, denoted as hIg). CH of class hIgG is suitable, and CH of any one of subclasses such as hIgG1, hIgG2, hIgG3, and hIgG4 belonging to class hIgG can be used. Moreover, human-type chimeric antibody CL may be any CL, as long as it belongs to hIg, and CL of class κ or CL of class λ can be used.

Examples of non-human animals include mice, rats, hamsters, and rabbits. Furthermore, it is known that coexistence of IgG inhibits MVM or PPV aggregation.

Examples of antibody fragments include peptides including Fab, F(ab')2, Fab', scFv, diabody, dsFv and CDR.

Fab is an antibody fragment having a molecular weight of about 50,000 and antigen-binding activity, wherein about a half portion on the N-terminal side of the H chain and the whole L chain are linked by a disulfide bond, among fragments obtained by treating an IgG antibody molecule with a protease, papain, (which cleaves the H chain at amino acid residue 224). Fab can be produced by treating an antibody with a protease, papain, or inserting DNA encoding Fab of the antibody into an expression vector for prokaryotes or an expression vector for eukaryotes, and then introducing the vector into prokaryotes or eukaryotes.

In the embodiments, the step of adjusting the pH of a solution contaminated with PCV to range from 3.8 to 7.0 is performed by measuring a pH of the solution contaminated with PCV and then adjusting the pH to range from 3.8 to 7.0.

When the protein concentration in a solution contaminated with PCV is less than 0.10 w/v%, the pH of the solution contaminated with PCV is preferably adjusted to up to 6.0.

When a solution contaminated with PCV has a protein concentration ranging from 0.10 to 1.5 w/v%, the pH of the solution contaminated with PCV is adjusted to range from pH5.5 to 6.5.

Adjustment of pH can be performed using a base, an acid, and a buffer. Examples of a base include sodium hydroxide. As an acid, any of inorganic acid and organic acid may be used and an example of the same is hydrochloric acid. As a buffer, examples thereof include an acetate buffer, a citrate buffer and a phosphate buffer.

In view of virus removal, the pH-adjusted solution contains glycine.

Glycine concentration in the pH-adjusted solution ranges from 0.010 to 0.40 M, preferably 0.20 to 0.35 M.

The solution contaminated with PCV has a protein concentration ranging from 0.10 to 1.5 w/v% and the glycine concentration in the pH-adjusted solution is adjusted to range from preferably 0.010 to 0.40 M, and preferably 0.010 to 0.30 M.

In the solution contaminated with PCV containing a protein, the glycine concentration or the pH of the pH-adjusted solution are also adjusted as appropriate based also on the protein concentration.

In the embodiments, through pH adjustment, PCV can be effectively removed by filtration without having any effect on the properties and the yield of a target protein. Moreover, through adjustment of the glycine concentration within a suitable range, PCV can be removed more effectively.

This may be because, but is not restrained by the principle, PCV aggregation takes place in a sample contaminated therewith, the size of aggregated PCV increases to a size larger than that of a target protein molecule contained in the sample, and thus PCV is efficiently removed.

In the embodiments, the step of treating the pH-adjusted solution with a filtration membrane having an LRV for bacteriophage PP7 of 4 or more is performed by passing the pH-adjusted solution through the filtration membrane having an LRV for bacteriophage PP7 of 4 or more.

In the embodiments, through treatment with a filtration membrane having an LRV for bacteriophage PP7 of 4 or more, PCV can be effectively removed.

The term "filtration membrane having an LRV for bacteriophage PP7 of 4 or more" refers to a membrane having an LRV of 4 or more when the filtration membrane to be used is loaded with a 50L/m$^2$ solution containing bacteriophage PP7. As a solution containing bacteriophage PP7, a 1 mg/mL BSA solution (PBS buffer, pH 7.4) containing bacteriophage PP7 at a concentration of $10^7$ pfu/mL is used.

The shape of a filtration membrane to be used herein is not particularly limited, and an example thereof may be a plain membrane, or a hollow fiber membrane. When a plain membrane is used, filtration may be performed with a single plain membrane or overlaid multiple plain membranes.

Filtration is performed by passing a pH-adjusted solution through a filtration membrane having an LRV for bacteriophage PP7 of 4 or more, thereby treating the pH-adjusted solution with the filtration membrane having an LRV for bacteriophage PP7 of 4 or more.

Regarding a filtration method, filtration may be performed by full-flow filtration or cross-flow filtration.

Before treatment with a filtration membrane having an LRV for bacteriophage PP7 of 4 or more, preliminary filtration may be performed with a membrane having an average pore size of greater than 20 nm.

Specific examples of such a filtration membrane having an LRV for bacteriophage PP7 of 4 or more include Planova™ 15N (Asahi Kasei Medical) and Planova™ 20N (Asahi Kasei Medical) made of cellulose, Planova™ BioEX (Asahi Kasei Medical) and Pegasus SV4 (Pall) made of hydrophilic PVDF, as well as Virosart CPV (Sartorius) and Viresolve Pro (Millipore) made of hydrophilic PES.

As a filtration membrane having an LRV for bacteriophage PP7 of 4 or more, in the case of virus removal membrane made of cellulose, a preferable membrane can also be specified based on the average pore size of open pores of a virus removal membrane. An average pore size of open pores of a virus removal membrane is, for example, 13 nm or more and 21 nm or less.

The average pore size of open pores can be calculated by the following formula with reference to the method described in International Publication No. WO2015/156401.

$$\text{Average pore size (nm)} = 2 \times 103 \times \sqrt{(V \cdot d \cdot \mu / P \cdot A \cdot Pr)}$$

Here "V" denotes the water permeation quantity (mL/min), "d" denotes membrane thickness ($\mu$m), "$\mu$" denotes the viscosity of water (cp), "P" denotes pressure difference (mmHg), "A" denotes membrane surface area (cm$^2$), and "Pr" denotes porosity (%).

The water permeation quantity is measured as follows. Ten (10) strings are tied in a bundle, a module is prepared in such a manner that the effective length is 16 cm, one end of the thus obtained module is closed, and then pressure of 200 mmHg is applied to the other end, followed by water passage at 37°C. The amount of water that comes out from the membrane via permeation is measured as the water permeation quantity.

A membrane surface area is calculated by measuring an inside diameter in a dry state. Moreover, the term "membrane thickness" refers to a membrane thickness in a dry state.

Porosity is calculated by the following formula.

$$\text{Porosity (\%)} = (1 - \rho a / \rho p) \times 100$$

The apparent density of hollow fiber pa is calculated by the following formula, and "pp" denotes cellulose density (g/cm$^3$).

$$\text{Apparent density of hollow fiber (g/cm}^3\text{)} = Wd/Vw = 4Wd/\pi l(Do^2 - Di^2)$$

Here, "Wd" denotes the absolute dry weight of hollow fiber (g), "Vw" denotes the apparent volume of hollow fiber (cm$^3$), "l" denotes the length of hollow fiber (cm), "Do" denotes the outside diameter of hollow fiber (cm), and "Di" denotes the inside diameter of hollow fiber (cm) .

In the embodiments, the term "LRV" refers to a Log Reduction Value (virus removal coefficient), which is a degree of virus reduction represented by logarithm and is also referred to as a removal coefficient.

LRV is found by the following formula.

$$LRV = \text{Log}[(V_1 \times T_1))]/[(V_2 \times T_2))]$$

$V_1$: Volume of a sample before the step of virus removal treatment
$T_1$: Viral load (titer) before the step of virus removal treatment
$V_2$: Volume of a sample after the step of virus removal treatment
$T_2$: Viral load (titer) after the step of virus removal treatment

The viral load of bacteriophage PP7 can be measured by a Plaque assay method, but the method is not particularly limited thereto.

The plaque assay method can be performed according to the method described in Journal of Pharmaceutical Science and Technology 2008; Supplement Volume 62 No. S-4.

A solution sample containing bacteriophage PP7 is serially diluted, each sample is mixed with *Pseudomonas aeruginosa,* and then soft agar is added. The mixed solution is poured onto an agar plate and then solidified, followed by 1 day of culturing at 37°C. On the next day, the number of plaques is counted visually, and then the concentration of phage (pfu: plaque forming unit)/mL contained in the original solution is calculated by the following formula.

$$\text{(Number of plaques} \times \text{dilution rate) pfu/amount of}$$

$$\text{sample (mL) pfu/mL}$$

In the embodiments, PCV removal by the treatment method in the embodiments can be confirmed based on LRV as an index.

An LRV for PCV can be found by measuring the viral load of PCV in the above formula.

The viral load of PCV can be measured by quantitative PCR (Quantitative polymerase chain reaction: Q-PCR), but the method is not particularly limited thereto.

A quantitative PCR method can be performed according to Yang et al.'s method (described in Non-Patent Literature 1). In this case, PCV-1 TM Fwd.:
AGAAAGGCGGGAATTGAAGATAC (SEQ ID NO: 1) and PCV-1 TM Rev.: CACACCCCGCCTTCAGAA (SEQ ID NO: 2) are used as primers, and TaqMan probe: 6FAM-CGTCTTTCGGCGCCATCTGTAACG-TAMRA (SEQ ID NO: 3) is used as a probe.

In the embodiments, when an LRV for PCV is measured and found to be 2 or more, this can be concluded as effective removal of PCV.

In the embodiments, a pH-adjusted solution to be applied to a filtration membrane having an LRV for bacteriophage PP7 of 4 or more is preferably treated at 50 to 100 $L/m^2$, which is the amount of the solution to be filtered per area of the virus removal membrane.

Through treatment at 50 to 100 $L/m^2$, the sample can be effectively filtered without clogging of the virus removal membrane with proteins and the like.

The area of a virus removal membrane is calculated as an area of a filtration surface (surface on the primary side). In the case of a plain membrane, the area is found by multiplying the length of one side times the length of another side. In the case of a hollow fiber membrane, the area is an area of the inner surface within the hollow fiber.

The amount of a solution to be filtered can be regulated by the time for filtration under the conditions of a constant filtration pressure.

According to the method for treating a solution contaminated with PCV of the embodiments, the pH of the solution containing PCV is adjusted to be a predetermined pH, so as to cause viral aggregation of PCV smaller in size than the pore size of a general virus removal membrane, so that PCV can be removed using the virus removal membrane. Furthermore, according to the method for treating a solution contaminated with PCV of the embodiments, an effect can be obtained such that safety can be ensured for a solution that can be contaminated with PCV (a case of being not contaminated with PCV is included).

When a solution contaminated with PCV in the embodiments contains a protein, through treatment with a virus removal membrane, both safety and protein permeability can be satisfied simultaneously. Furthermore, when a solution contaminated with PCV of the embodiments contains no protein, through treatment with a virus removal membrane, PCV can be removed.

Specifically, according to the treatment method of the embodiments, PCV can be removed from a solution containing PCV through filtration with a membrane having an LRV of 4 or more. Moreover, through filtration of a solution that can be contaminated with PCV, safety can be ensured. Furthermore, according to the treatment method of the embodiments, regardless of the presence or the absence of proteins, PCV can be removed by the method, as well as both high permeability and safety can be satisfied simultaneously in the case of a protein-containing solution.

As described in the following Examples, in an aspect, the recovery rate of a target protein was 80% or more according to the treatment method of the embodiments. Specifically, according to the treatment method of the embodiments, products with high safety, from which PCV has been removed, can be provided.

Examples

The present invention will be described specifically with reference to the following examples to help the understanding of the present invention.

(Example 1) Examination of optimum pH under glycine buffer conditions

With the use of a 0.30 M glycine buffer, changes in capacity of removing PCV under the conditions of pH3.0 to 8.0 were confirmed based on LRV.

1. Materials and uses

- Viral strain (PCV): As a virus source of PCV, a culture solution of the porcine kidney-derived established cell line (PK (15) cells: ATCC No. CCL-33) was used. PK (15) cells, $1.0 \times 10^5$ cells/mL, were seeded and cultured (37°C, 5%$CO_2$) in 5% fetal bovine serum-containing Dulbecco's modified Eagle's medium. The culture supernatant on day 4 of culturing was used as a virus source. The virus source was stored at -80°C. The genome concentration of the virus source was 10.17 Log copies/mL.
- Glycine buffer: 27.0 g of glycine (purchased from the manufacturer: WAKO) was added to 1.2 L of pure water, and then the solution was stirred using a stirrer for dissolution. After glycine was completely dissolved, 0.50 M hydrochloric acid or sodium hydroxide aqueous solution was added to the solution while stirring with a stirrer, so as to adjust the pH to a target pH.
- Filtration membrane used herein: Planova15N (Asahi Kasei Medical Co., Ltd. Hereinafter, referred to as "P-15"), Planova20N (Hereinafter, referred to as "P-20"), and Planova35N (Hereinafter, referred to as "P-35") were used. Each membrane surface area was 0.001 $m^2$. An LRV for PP7 was >6.5 in the case of P-15 and P-20, and was 0 in the case of P-35.

- Reagent for PCR: QuantiTect Probe PCR Kit, Purchased from QIAGEN
- IgG: Blood donation Venoglobulin IH (Japan Blood Products Organization), concentration: 10%, biological species: human

2. Methods

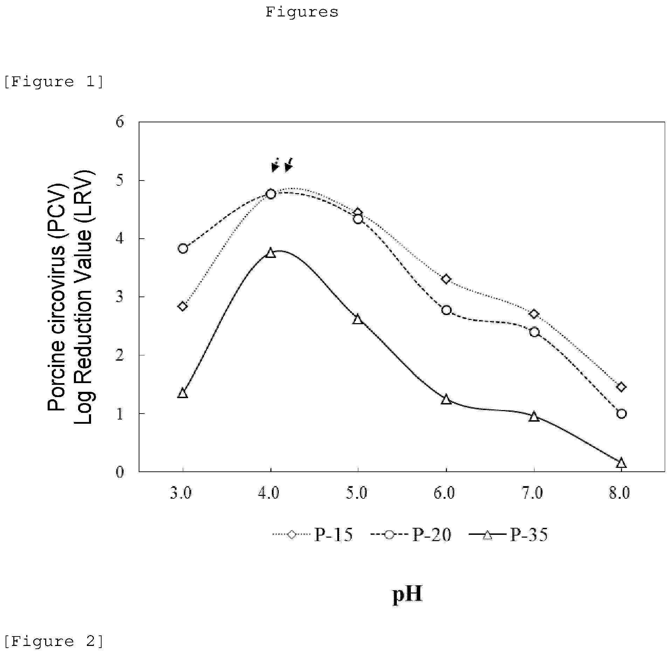

One (1) mL each of PCV (corresponding to a 1/100 amount) was added to a 0.30M glycine buffer of 0.1 L having pH adjusted to range from pH3.0 to 8.0. These samples were subjected to dead-end filtration using the filtration membrane under the conditions of filtration pressure of 0.08 MPa, and the amount of solution treated of 0.03 L.
Filtration membranes used herein were P-15 and P-20 as examples and P-35 as a comparative example. PCV genome amounts before and after filtration were measured by the Q-PCR method, thereby calculating the LRV.
Results are shown in Figure 1 and Table 1.
3. Capacity of removing PCV was determined based on a log reduction value (LRV) calculated by the following formula.

$$\mathrm{LRV} = \mathrm{Log} \; [(V_1 \times T_1))]/[(V_2 \times T_2))]$$

$V_1$: PCV genome volume of a sample before the step of virus removal treatment
$T_1$: PCV genome amount before the step of virus removal treatment
$V_2$: PCV genome volume of a sample after the step of virus removal treatment
$T_2$: PCV genome amount after the step of virus removal treatment

4. Results

In the presence of a glycine buffer having the above concentration, it was confirmed that PCV was removed using a virus removal membrane in a pH dependent manner. The LRVs of P-15 and P-20 at pH4.0 were each ≥5. 0 (Table 1 · Figure 1). The LRV of P-35 at pH4.0 was 3.8. In addition, a spot pointed by arrows in Figure 1 indicate decreases in virus the detection limit or lower.

[Table 1]

| pH | 3.0 | 4.0 | 5.0 | 6.0 | 7.0 | 8.0 |
|------|-----|------|-----|-----|-----|-----|
| P-15 | 2.8 | ≥5.0 | 4.4 | 3.3 | 2.7 | 1.5 |
| P-20 | 3.8 | ≥5.0 | 4.3 | 2.8 | 2.4 | 1.0 |
| P-35 | 1.4 | 3.8 | 2.6 | 1.3 | 1.0 | 0.2 |

(Example 2) Examination of optimum glycine concentration

In this example, changes in capacity of removing PCV under the conditions of 0.10 to 2.0 M glycine buffers (pH4.0) were confirmed. Except the conditions of the pH of glycine buffers fixed at pH 4 and glycine concentrations ranging from 0.1 to 2.0 M, each sample was prepared in the same manner as in Example 1. Each sample was filtered by the same technique as in Example 1, and then the LRV was calculated. Results are depicted in Figure 2 and Table 2.

[Table 2]

| Glycine concentration (M) | 0 | 0.10 | 0.30 | 0.50 | 2.0 |
|---|---|---|---|---|---|
| P-15 | ≥4.8 | ≥4.5 | ≥5.0 | 4.2 | 4.1 |
| P-20 | ≥4.8 | ≥4.5 | ≥5.0 | 3.9 | 2.2 |
| P-35 | ≥4.8 | 4.5 | 3.8 | 2.5 | 1.2 |

In both cases of using P-15 and P-20, an LRV when the glycine concentration was 0.10 M, and an LRV when the same was 0.30 M were ≥4.5 and ≥5.0, respectively. When the glycine concentration was 0.50 M, LRVs were 4.2 and 3.9, respectively. When P-35 was used, an LRV when the glycine concentration was 0.10M, and an LRV when the same was 0.30 M were 4.5 and 3.8, respectively (Table 2). Note that spots pointed by arrows in Figure 2 indicate decreases in virus to the detection limit or lower.
Under conditions of 0.10 M glycine buffer (pH4.0), the virus removal capacity was high under the conditions of a low IgG concentration.

(Example 3) Examination of optimum pH under conditions of 0.10% IgG and 0.10 M glycine

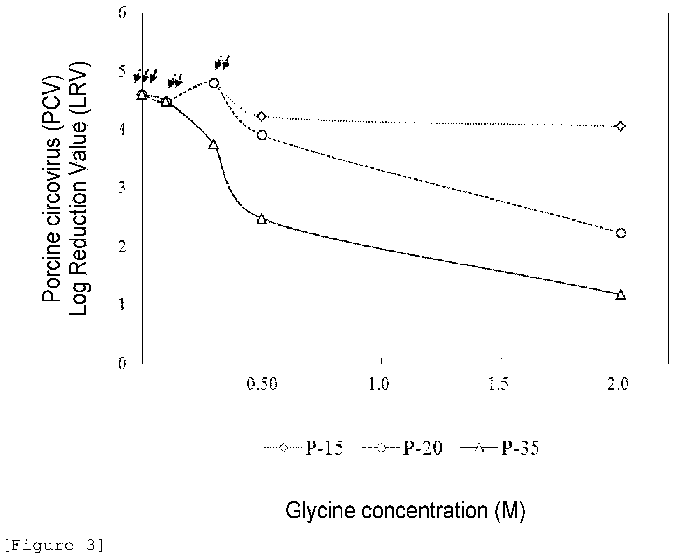

In this example, the effect of pH on PCV removal under the conditions of 0.10% IgG and 0.10 M glycine was confirmed. Except for adding 0.10% IgG to 0.10 M glycine buffers (pH3.5 to 6.0), each sample was prepared in the same manner as in Example 1. Each sample was filtered using P-15 under the same conditions as in Example 1, thereby calculating the LRV. Results are depicted in Figure 3 and Table 3.

[Table 3]

| pH | 3.5 | 4.0 | 4.5 | 5.0 | 6.0 |
|---|---|---|---|---|---|
| P-15 | 1.7 | 2.6 | 2.9 | 3.2 | 3.7 |

(Example 4) Examination of optimum glycine concentration under conditions of 0.10% IgG and pH6.0

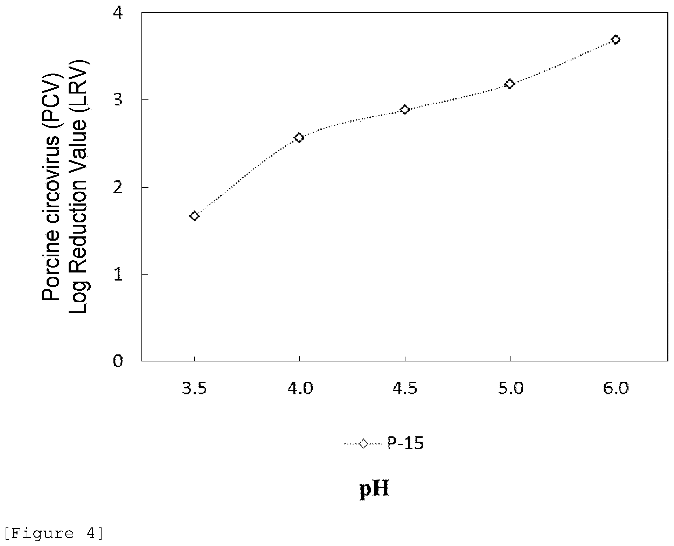

In this example, the effect of glycine concentrations on PCV removal under the conditions of 0.10% IgG and pH6.0 was confirmed. Except for adding 0.10% IgG to 0.10 to 0.50 M glycine buffers (pH6.0), each sample was prepared in the same manner as in Example 1. Each sample was filtered using P-15 and P-20 under the same conditions as in Example 1, thereby calculating the LRV. Results are depicted in Fig 4 and Table 4.

[Table 4]

| Glycine concentration (M) | 0.10 | 0.30 | 0.50 |
|---|---|---|---|
| P-15 | 3.7 | 3.7 | 3.9 |
| P-20 | 3.4 | 3.1 | 3.3 |

(Example 5) Examination of protein concentration under conditions of 0.30 M glycine and pH6.0

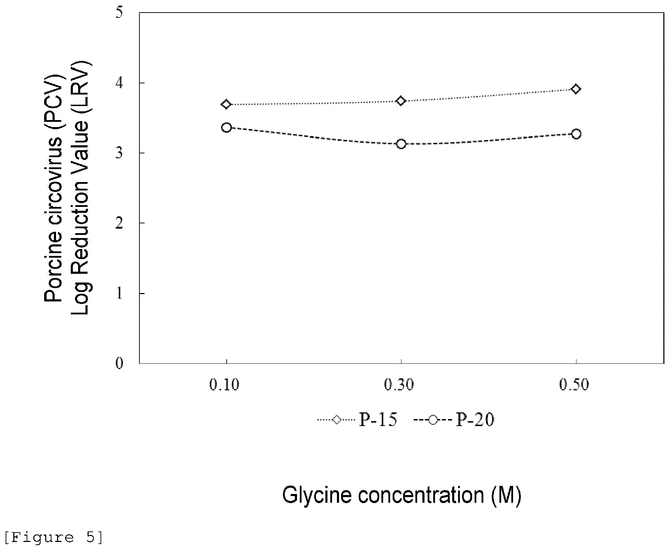

In this example, the effect of IgG concentration on PCV removal under the conditions of a 0.30 M glycine buffer (pH6) was confirmed. Except for adding 0.10 to 5.0% IgG to a 0.30 M glycine buffer (pH6), each sample was prepared in the same manner as in Example 1. Under the conditions of IgG concentration of 1.0%, two P-15 filters and two P-20 filters were used. Under the conditions of IgG concentrations other than this concentration, P-20 was used. Each sample was filtered under the same conditions as in Example 1, thereby calculating the LRV. Results are depicted in Figure 5 and Table 5.

[Table 5]

| IgG concentration (%) | 0.10 | 0.50 | 1.0 | 1.5 | 2.0 | 5.0 |
|---|---|---|---|---|---|---|
| P-15 | NT | NT | 2.2/2.3 | NT | NT | NT |
| P-20 | 3.1 | 2.5 | 2.3/2.3 | 1.9 | 1.0 | 1.0 |

(Example 6) Protein recovery rate as a result of filtration under the conditions of 1% IgG-containing 0.30 M glycine (pH6.0)

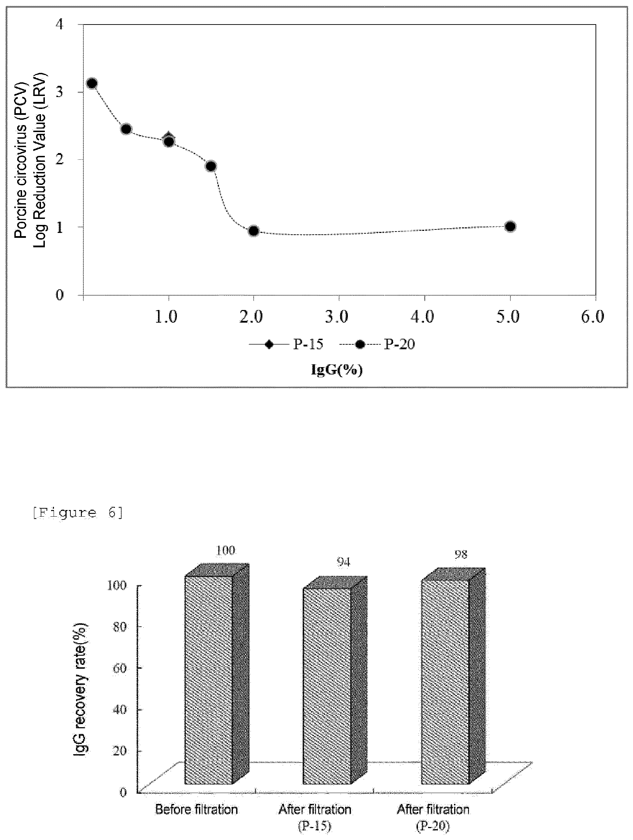

In this example, after filtration with P-15 and P-20 under the conditions of 1% IgG-containing 0.30 M glycine (pH6.0), IgG recovery rates were confirmed. IgG recovery rate after filtration was measured using an absorptiometer (A280 nm). When P-15 and P-20 were loaded with samples having fluid volumes of 150 mL and 53 mL, the recovered fluid volumes after filtration were 147.6 mL and 50.7 mL. The recovery rate (%) indicates the percentage of the amount of IgG in a sample after filtration relative to the loaded amount of IgG in the sample designated as 100%. The recovery rate after filtration with P-20 was 98%, and the recovery rate after filtration with P-15 was 94% (Figure 6).

Industrial Applicability

The method of the present invention is useful in production of particularly blood plasma protein fraction preparations and biopharmaceuticals.

Sequence Listing Free Text

SEQ ID NO: 1 represents the nucleotide sequence of a primer (PCV-1 TM Fwd.).
SEQ ID NO: 2 represents the nucleotide sequence of a primer (PCV-1 TM Rev.).
SEQ ID NO: 3 represents the nucleotide sequence of a probe (TaqMan probe).

## Claims

1.  A method for treating a solution contaminated with porcine circovirus, comprising the steps of:

    adjusting the pH of a solution contaminated with porcine circovirus to range from 3.8 to 7.0, wherein the pH-adjusted solution contains glycine in an amount of 0.010 to 0.40 M, and
    treating the pH-adjusted solution with a filtration membrane having a log removal value (LRV) for bacteriophage PP7 of 4 or more, and wherein the LRV for porcine circovirus is 2 or more,
    wherein said solution contains a protein at a protein concentration ranging from 0.10 to 1.5 w/v %,
    said filtration membrane having an LRV for bacteriophage PP7 of 4 or more is a membrane having an LRV of 4 or more when the filtration membrane to be used is loaded with a 50 $L/m^2$ solution containing bacteriophage PP7, and said solution containing bacteriophage PP7 is a 1 mg/mL BSA solution (PBS buffer, pH 7.4) containing bacteriophage PP7 at a concentration of $10^7$ pfu/mL.

2.  The method for treating a solution contaminated with porcine circovirus according to claim 1, wherein the solution contains 0.20 to 0.35 M glycine.

3.  The method for treating a solution contaminated with porcine circovirus according to any one of claims 1 or 2, wherein the protein is IgG.

4.  The method for treating a solution contaminated with porcine circovirus according to any one of claims 1 to 3, wherein the pH-adjusted solution is treated at 50 to 100 $L/m^2$.

## Patentansprüche

1.  Verfahren zur Behandlung einer Lösung, die mit porcinem Circovirus kontaminiert ist, umfassend die Schritte:

    Einstellen des pH-Werts einer Lösung, die mit porcinem Circovirus kontaminiert ist, in einem Bereich von 3,8 bis 7,0, wobei die Lösung mit dem eingestellten pH-Wert Glycin in einer Menge von 0,010 bis 0,40 M enthält; und
    Behandeln der Lösung mit dem eingestellten pH-Wert mit einer Filtrationsmembran, die einen logarithmischen Reduktionswert (LRV) für Bakteriophage PP7 von 4 oder mehr aufweist, wobei der LRV für porcines Circovirus 2 oder mehr beträgt;
    wobei die Lösung ein Protein in einer Proteinkonzentration im Bereich von 0,10 bis 1,5% (w/v) enthält;
    wobei die Filtrationsmembran, die einen LRV für Bakteriophage PP7 von 4 oder mehr aufweist, eine Membran ist, die einen LRV von 4 oder mehr aufweist, wenn die zu verwendende Filtrationsmembran mit einer 50-l/m$^2$-Lösung, die Bakteriophage PP7 enthält, beladen ist und die Lösung, die Bakteriophage PP7 enthält, eine 1-mg/ml-BSA-Lösung ist (PBS-Puffer, pH 7,4), die Bakteriophage PP7 in einer Konzentration von 10$^7$ pfu/ml enthält.

**2.** Verfahren zur Behandlung einer Lösung, die mit porcinem Circovirus kontaminiert ist, gemäß Anspruch 1, wobei die Lösung 0,20 bis 0,35 M Glycin enthält.

**3.** Verfahren zur Behandlung einer Lösung, die mit porcinem Circovirus kontaminiert ist, gemäß einem der Ansprüche 1 oder 2, wobei es sich bei dem Protein um IgG handelt.

**4.** Verfahren zur Behandlung einer Lösung, die mit porcinem Circovirus kontaminiert ist, gemäß einem der Ansprüche 1 bis 3, wobei die Lösung mit dem eingestellten pH-Wert mit 50 bis 100 l/m$^2$ behandelt wird.

**Revendications**

**1.** Procédé pour le traitement d'une solution contaminée avec du circovirus porcin, comprenant les étapes de :

ajustement du pH d'une solution contaminée avec du circovirus porcin dans un intervalle de 3,8 à 7,0, où la solution à pH ajusté contient de la glycine dans une quantité de 0,010 à 0,40 M, et
le traitement de la solution à pH ajusté avec une membrane de filtration ayant un log de valeur d'élimination (LRV) pour un bactériophage PP7 de 4 ou supérieur, et/ou le LRV pour le circovirus porcin est de 2 ou supérieur, où ladite solution contient une protéine à une concentration en protéine de 0,10 à 1,5 en % masse/volume, ladite membrane de filtration présentant un LRV pour un bactériophage PP7 de 4 ou supérieur est une membrane ayant un LRV de 4 ou supérieur lorsque la membrane de filtration à utiliser est chargée avec une solution à 50 l/m$^2$ contenant un bactériophage PP7, et ladite solution contenant un bactériophage PP7 est une solution de BSA à 1 mg/ml (tampon PBS, pH 7,4) contenant un bactériophage PP7 à une concentration de 10$^7$ pfu/ml.

**2.** Procédé pour le traitement d'une solution contaminée avec du circovirus porcin selon la revendication 1, où la solution contient de 0,20 à 0,35 M de glycine.

**3.** Procédé pour le traitement d'une solution contaminée avec du circovirus porcin selon l'une quelconque des revendications 1 ou 2, où la protéine est IgG.

**4.** Procédé pour le traitement d'une solution contaminée avec du circovirus porcin selon l'une quelconque des revendications 1 à 3, où la solution à pH ajusté est traitée à de 50 à 100 l/m$^2$.

Figures

[Figure 1]

[Figure 2]

[Figure 3]

[Figure 4]

[Figure 5]

[Figure 6]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012176876 A
- JP 2004339079 A
- JP 2006151840 A

- WO 2015156401 A
- JP 2016156506 A

### Non-patent literature cited in the description

- **HONGO-HIRASAKI T. et al.** Removal of small viruses (parvovirus) from IgG solution by virus removal filter Planova 20N. *JOURNAL OF MEMBRANE SCIENCE,* 06 December 2005, vol. 278 (1-2), 3, , 9
- **FURUYA K. et al.** Implementation of a 20-nm pore-size filter in the plasma-derived Factor VIII manufacturing process. *VOX SANGUINIS,* 16 May 2006, vol. 91 (2), 119-125

- **YAN B et al.** Porcine Circovirus (PCV) removal by Q sepharose fast flow chromatography. *Biotechnol. Prog.,* 2013, vol. 29, 1464-1471
- *Journal of Pharmaceutical Science and Technology,* 2008, vol. 62 (4